# EUROPÄISCHE PATENTSCHRIFT

(11) **EP 2 020 221 B1**
(45) Veröffentlichungstag und Bekanntmachung des Hinweises auf die Patenterteilung: **29.02.2012**
(21) Anmeldenummer: 07110571.2
(22) Anmeldetag: 19.06.2007
(51) Int. Cl.: A61K 8/02, A61K 8/04, A61K 8/36, A61K 8/37, A61Q 17/04, A61Q 19/00, A61Q 19/10, A61K 9/12, A61K 9/127

(54) **DMS (Derma Membrane Structure) in Schaum-Cremes**
DMS (Derma Membrane Structure) in foaming creams
DMS (Derma Membrane Structure) dans des crèmes moussantes

(43) Veröffentlichungstag der Anmeldung: 04.02.2009
(73) Patentinhaber: Neubourg Skin Care GmbH & Co. KG, 48268 Greven (DE)
(72) Erfinder: Neubourg, Thomas, Dr., 59368 Werne (DE)
(74) Vertreter: Ehlich, Eva Susanne

(56) Entgegenhaltungen:
- EP-A- 1 352 639
- WO-A-03/017968
- FR-A- 2 825 629
- DATABASE WPI Week 200482 Derwent Publications Ltd., London, GB; AN 2004-826805 XP002463466 & JP 2004 331610 A (TAIYO KAGAKU KK) 25. November 2004 (2004-11-25)
- YU A SHCHIPUNOV: "Phase Behavior of Lecithin at the Oil/Water Interface", LANGMUIR, AMERICAN CHEMICAL SOCIETY, WASHINGTON, DC, US, vol. 12, no. 26, 1 January 1996 (1996-01-01), pages 6443-6445, XP007914535, ISSN: 0743-7463, DOI: DOI:10.1021/LA960082Y

## Beschreibung

### Technisches Gebiet der Erfindung

Die vorliegende Erfindung betrifft kosmetische und dermatologische Schaumformulierungen, insbesondere Schaumcremes, auf der Basis von Emulsionen insbesondere vom Typ Öl-in-Wasser, wobei die Ölphase mindestens eine membranbildende Substanz umfasst, die in der Schaumformulierung eine lamellar angeordnete Membran ausbildet.

### Hintergrund der Erfindung

### 1. Emulsionen:

Unter Emulsion versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z.B. Milch). Der Grundcharakter einer O/W-Emulsion ist durch das Wasser geprägt. Bei einer Wasser-in-Öl-Emulsion (W/O-Emulsion, z.B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (liphophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilien Molekülteil in ionische (anionische, kationische und amphotere) und nicht-ionische untergliedert werden:
- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären AmmoniumVerbindungen.
- Der hydrophile Molekülteil nicht-ionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer herkömmlichen Emulsion ist die richtige Auswahl der Emulgatoren. Dabei sind die Charakteristika aller im System enthaltenen Stoffe zu berücksichtigen. Betrachtet man z.B. Hautpflegeemulsionen, so können polare Ölkomponenten wie z. B. UV-Filter zu Instabilitäten führen. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar, welche in den unterschiedlichsten Anwendungsgebieten eingesetzt werden. So stehen für die Pflege der Haut, insbesondere für die Rückfettung trockener Haut, eine Vielzahl von Produkten - wie Lotionen und Cremes - zur Verfügung. Ziel der Hautpflege ist es, den durch tägliches Waschen verursachten Fett- und Wasserverlust der Haut auszugleichen. Außerdem sollen Hautpflegeprodukte vor Umwelteinflüssen - insbesondere vor Sonne und Wind - schützen und die Hautalterung verzögern.

Kosmetische Emulsionen werden auch als Desodorantien verwendet. Solche Formulierungen dienen dazu, Körpergeruch zu beseitigen, der entsteht, wenn der an sich geruchlose frische Schweiß durch Mikroorganismen zersetzt wird.

Auch zur Reinigung der Haut und Hautanhangsgebilde finden Emulsionen in Form von Reinigungsemulsionen Verwendung. Sie werden meist der Gesichtsreinigung und insbesondere zur Entfernung von dekorativer Kosmetik eingesetzt. Derartige Reinigungsemulsionen haben - im Gegensatz zu anderen Reinigungszubereitungen wie beispielsweise Seife - den Vorteil, besonders hautverträglich zu sein, da sie in ihrer lipophilen Phase pflegende Öle und/oder unpolare Wirkstoffe - wie z.B. Vitamin E - enthalten können.

### 2. Emulgatorfreie Emulsionen

Seit Jahrzehnten bilden herkömmliche Emulgatoren die Grundlage für die Entwicklung von Hautpflegepräparaten. Emulgatoren wurden als Hilfsstoffe zur Herstellung und vor allem zur Stabilisierung von Emulsionen eingesetzt. In jüngerer Zeit gibt es Hinweise, dass die Verwendung von Emulgatoren in Hautpflegepräparaten z.B. bei empfindlicher Haut zu Problemen führen kann, da die Emulgatoren typischerweise die Integrität der natürlichen Hautbarriere stören und es so bei der Hautreinigung zu einem Verlust von natürlichen Barrierestoffen der Haut kommen kann. Der Verlust der natürlichen Barrierestoffe kann erhöhte Rauigkeit, trockene Haut, Rissigkeit und Abnutzungsekzeme verursachen.

Weiterhin führt die Verwendung von Emulgatoren normalerweise dazu, dass die lamellaren Strukturen der Lipidbarriere in vesikuläre Strukturen, wie z.B. Liposomen umgewandelt werden. Diese Vesikel "zerstören" zumindest einen Teil der Barriereschicht der Haut und erhöhen dadurch lokal die Durchlässigkeit der Barriereschicht-Membran. Durch diese Öffnung der Barriereschicht der Haut wird zumindest vorübergehend der transepidermale Wasserverlust (TEWL) erhöht und gleichzeitig kommt es zu einer Abnahme des Feuchtigkeitsbindevermögens der Haut. Bei kontinuierlicher Anwendung von Pflegepräparaten mit herkömmlichen Emulgatoren kann es sogar dazu führen, dass die Haut nicht in der Lage ist, ihre Schutzfunktion aufrecht zu erhalten.

Eine besondere Form der Emulsion stellen emulgatorfreie Emulsionen dar. Diese sind frei von Emulgatoren im engeren Sinne, d.h. von amphiphilen Substanzen mit niedrigen Molmassen (Molmasse < 5000), die in geeigneten Konzentrationen Micellen und/oder andere flüssigkristalline Aggregate ausbilden können.

Ein Beispiel für emulgatorfreie Emulsionen sind Pickering-Emulsionen. Pickering Emulsionen sind feststoffstabilisierte Emulsionen, in denen die feinstverteilten Feststoffteilchen die Emulsion stabilisieren, so dass weitestgehend auf herkömmliche Emulgatoren verzichtet werden kann. Dabei kommt es zu einer Anreicherung des festen Stoffes an der Phasengrenze Öl/Wasser in Form einer Schicht, wodurch ein Zusammenfließen der dispersen Phase verhindert wird. Solche geeigneten Feststoffemulgatoren sind partikuläre anorganische oder organische Feststoffe, die sowohl von lipophilen als auch von hydrophilen Flüssigkeiten benetzbar sind. Bevorzugt werden in den Pickering Emulsionen z.B. Titandioxid, Zinkoxid, Siliziumdioxid, Fe₂O₃, Veegum, Bentonit oder Ethylcellulose als Feststoffe eingesetzt.

Jedoch können auch diese Feststoffemulgatoren bei empfindlicher Haut zu Reizungen führen oder sogar Allergien auslösen.

Es werden heute bereits Cremegrundlagen eingesetzt, die mit einer Vielzahl von natürlichen bzw. hautähnlichen Inhaltsstoffen arbeiten, die eine bessere Hautverträglichkeit insbesondere bei empfindlicher Haut versprechen. Dabei hat sich gezeigt, dass durch die Verwendung von hautähnlichen Inhaltsstoffen eine verbesserte Hautpflege erzielen lässt. So werden in diesen Cremegrundlagen z.B. einige Bestandteile der natürlichen Hautlipide wie zB. Triglyceride durch (pflanzliche) Caprylsäure/Caprinsäuretriglyceride , Squalen durch (pflanzliches) Squalan, Ceramide durch Ceramide 3 (aus Hefe), Cholesterol durch (pflanzliche) Phytosterole und Phospholipide durch (pflanzliche) Phospholipide ersetzt.

Bevorzugt wird bei diesem Konzept auf viele übliche Hilfsstoffe wie Duftstoffe, Farbstoffe, komedogene Lipide (z.B. Mineralöle), Konservierungsmittel und physiologisch bedenkliche Emulgatoren verzichtet, da diese potenziell sensibilisierend sind und zu Irritationen der Haut führen können.

Diese Formulierungen werden bevorzugt ohne herkömmliche Emulgatoren zubereitet, um die oben genannten Nachteile von herkömmlichen Emulgatoren zu vermeiden.

Ohne sich auf eine bestimmte Theorie zu stützen, wird angenommen, dass die besondere Wirkung dieser spezifisch zusammengesetzten Membranlipide mit der lamellaren Struktur zusammenhängt. Der Verzicht auf herkömmliche Emulgatoren verhindert, dass Micellen oder Vesikel gebildet werden, so dass die lamellare Struktur der ausgebildeten Membran in der Emulsion erhalten bleibt. Diese lamellare Struktur ist dem (physikalischen) Aufbau und der (chemischen) Zusammensetzung der natürlichen Haut nachempfunden.

Systeme, die auf spezifisch zusammengesetzte Membranlipide zurückgreifen, die eine lamellare Struktur der Membran aufweisen, sind unter dem Begriff "DMS®" (Derma Membrane Structure) in der Technik bekannt.

### 3. Schaumformulierungen

Eine besondere Anwendungsform kosmetischer und oder dermatologischer Emulsionen ist die Applikation als Schäume. Schaumformulierungen haben den Vorteil, sich leicht auf der Haut verteilen zu lassen. Die schaumige Konsistenz wird als angenehm empfunden und die Produkte hinterlassen in der Regel ein gutes Hautgefühl. Insbesondere wirkt sich aber auch die physikalische Struktur des Schaumes positiv auf die Hautschutzfunktion aus. Schäume sind komplizierte physikalische Gebilde, die einer besonderen Abstimmung der den Schaum bildenden Komponenten bedürfen. Schäume werden generell durch Versprühen einer Emulsionsformulierung oder einer wässrigen Tensid-(Stabilisator-)lösung erhalten. Beispielsweise wird die mit Treibgas versetzte Emulsion aus einem unter Druck stehenden Behältnis abgegeben (solche Systeme werden in der Literatur und Patentliteratur auch als Aerosolschäume bezeichnet). Dabei expandiert das unter Druck stehende Gemisch aus Emulsion und Treibgas und bildet die Schaumbläschen. Insbesondere kommt es zu einer Expansion der dispersen Ölphase, in welcher das öllösliche Gas gelöst ist. Schäume können aber auch mit Hilfe anderer Systeme erzeugt werden, wie beispielsweise Pumpsprays.

Abgestimmte Schaumformulierungen weisen eine stabile zwei- oder mehrphasige polydisperse Struktur bei der Applikation auf, die auf der Haut eine Netzstruktur, vergleichbar mit einer Membran bildet. Solche Netzstrukturen haben den Vorteil, dass diese eine Schutzfunktion, beispielsweise vor Kontakt mit Wasser ausbilden, jedoch einen ungehinderten Gasaustausch mit der Umgebung zulassen. Bei solchen Schäumen kommt es praktisch zu keiner Behinderung der *Perspiratio insensibiles* und keinem entsprechenden Wärmestau. Damit werden die positiven Eigenschaften einer Schutz- und Pflegefunktion mit einer unveränderten Hautatmung verbunden.

Bisher bekannte Schaumformulierungen enthalten herkömmliche Tenside/Emulgatoren, die für die Stabilisierung der Emulsion und für die darauf folgende Schaumstabilität sorgen.

Herkömmliche Emulgatoren bzw. Tenside werden jedoch, wie bereits zuvor diskutiert, immer wieder als Ursache für Unverträglichkeiten bei Hautpflegeprodukten genannt, wie z.B. eine Störung der Hautbarriere oder Mallorcaakne.

Daher besteht ein Bedarf an individuellen Hautpflegezusammensetzungen, die den Hautbedürfnissen besser gerecht werden, als herkömmliche Emulsionssysteme auf Emulgatorbasis und somit einen besseren Hautschutz und eine verbesserte Hautpflege bereitstellen.

Ein Einsatz von Cremegrundlagen, die eine lamellare Struktur in der Zusammensetzung aufweisen, ist in Schaumformulierungen bislang nicht beschrieben worden.

Aufgabe der vorliegenden Erfindung ist es, verbesserte Schaumformulierungen, insbesondere bessere Schaumcremes, bereitzustellen, die die oben genannten Nachteile der Formulierungen des Standes der Technik umgeht.

### Zusammenfassung der Erfindung

Die Anmelderin hat überraschenderweise erkannt, dass sich Emulsionen, die eine Ölphase und eine Wasserphase umfassen, wobei die Ölphase mindestens eine membranbildende Substanz umfasst, die in der Schaumformulierung eine lamellar angeordnete Membran ausbildet, als Basis für Schaumformulierungen eignen. Die vorliegende Erfindung betrifft daher eine Schaumformulierung gemäß dem beigefügten Anspruch 1.

Dabei werden die positiven Eigenschaften der Schaumformulierungen mit denen der Emulsionen, in denen die Ölphase mindestens eine membranbildende Substanz umfasst, die in der Schaumformulierung eine lamellar angeordnete Membran ausbildet, verbunden. Dabei lassen sich insbesondere Schaumformulierungen herstellen, die die positiven Eigenschaften des Schaums, nämlich die physikalische Struktur und die angenehme Anwendbarkeit mit einer guten Hautverträglichkeit kombinieren. Diese Eigenschaft erlaubt den Einsatz der Schaumformulierungen für kosmetische und dermatologische Formulierungen, die bei empfindlichen Hauttypen eingesetzt werden. Es wird dabei Verträglichkeit und Anwendungskomfort vorteilhaft miteinander verbunden Die für die Hautverträglichkeit wichtige lamellare Struktur der mindestens einen membranbildenden Substanz ist in Schaumformulierungen des Standes der Technik nicht berücksichtigt worden.

Dabei ist es nicht selbstverständlich, dass diese Emulsionen beim Verschäumen zu stabilen Schaumprodukten führt. Schäume werden, wie bereits erwähnt, beispielsweise durch Einarbeiten von Treibgasen in O/W-Emulsionssystemen erhalten. Verdampft bei dem Verschäumen das z.B. in der dispersen Ölphase gelöste Treibgas, bildet sich ein Schaum (Gas in Flüssigdispersion). Beim Verdampfen bzw. Expandieren des in der dispersen Ölphase gelösten Treibgases kommt es zu einer Dilatation der dispersen Ölphase. Es ist nun überraschend gefunden worden, dass es beim Verschäumen der erfindungsgemäßen Schaumformulierungen nicht zum Brechen der Zubereitung kommt und ein geeigneter Schaum ausgebildet wird.

Die Erfindung betrifft Schaumformulierungen, umfassend eine Ölphase und eine Wasserphase, wobei die Ölphase mindestens eine membranbildende Substanz umfasst, die in der Schaumformulierung eine lamellar angeordnete Membran ausbildet, die mindestens eine membranbildende Substanz ein Lipid umfasst, wobei das Lipid hydriertes Lecithin umfasst.

Bevorzugt betrifft die Erfindung Schaumformulierungen, die auf Basis natürlicher bzw. hautähnlicher Inhaltsstoffe arbeiten, die eine bessere Hautverträglichkeit ermöglichen.

Ferner betrifft die Erfindung die Verwendung der verstehend beschriebenen Schaumformulierungen auf Basis von Emulsionen als Träger für Wirkstoffe, als Hautpflegemittel oder als Hautreinigungsmittel. Die Schaumformulierung kann daher als Kosmetikum, Medizinprodukt oder Arzneimittel eingesetzt werden.

Außerdem umfasst die Erfindung ein Verfahren zur Herstellung der verstehend beschriebenen Schaumformulierungen basierend auf Emulsionen, in denen die Ölphase eine membranbildende Substanz umfasst, die in der Schaumformulierung eine lamellar angeordnete Membran ausbildet. Das Verfahren umfasst die Schritte:
a) Herstellen einer Emulsion bevorzugt vom Typ Öl in Wasser
b) Abfüllen der Emulsion und Treibgas in einen Druckbehälter, oder
c) Abfüllen der Emulsion in einen anderen als einen Druckbehälter, der bei Abgabe der Emulsion einen Schaum erzeugt.

### Detaillierte Beschreibung der Erfindung

Gemäß der vorliegenden Erfindung sind Schaumformulierungen Formulierungen, insbesondere Emulsionen, die zur Erzeugung von Schaum sinnfällig hergerichtet sind. Die Formulierungen können insbesondere entweder mit Treibgas in einen Druckbehälter abgefüllt sein oder ohne Treibgas in einen anderen Behälter als einen Druckbehälter abgefüllt sein, der es ermöglicht, bei Abgabe der Formulierung/Emulsion einen Schaum zu erzeugen. Pumpspraybehälter können beispielsweise ebenfalls verwendet werden.

In einer bevorzugten Ausführungsform ist die Schaumformulierung eine Schaumcreme.

Gemäß der vorliegenden Erfindung sind im Wesentlichen emulgatorfreie Emulsionen solche Emulsionen, die nicht mehr als 1,5 Gew.-%, bevorzugt nicht mehr als 1,0 %, mehr bevorzugt nicht mehr als 0,5 % herkömmlicher Emulgatoren enthalten. Gemäß der Erfindung sind emulgatorfreie Emulsionen solche, die keine herkömmlichen Emulgatoren enthalten.

Gemäß der vorliegenden Erfindung ist eine membranbildende Substanz, die eine lamellar angeordnete Membran ausbildet, eine Substanz, die zugleich einen hydrophilen sowie einen hydrophoben Molekülrest aufweist. Bevorzugt sind Substanzen wie z.B. (Mono-, Di-, Tri-)-Glyceride, Lecithine, Sphingolipide, Phospholipide, Ceramide, Cholesterin, Squalen, Squalan, Fettalkohole, Fettsäuren sowie deren Mono- und/oder Diester, Sterole, etc.

Gemäß der vorliegenden Erfindung ist eine lamellar angeordnete Membran so angeordnet, dass sie einen Schichtaufbau besitzt, derart, dass die jeweils obere Schicht der Substanz zu einer unteren Schicht der Substanz zueinander ausgerichtet ist. Die Ausrichtung der einzelnen Substanzschichten erfolgt zueinander unabhängig von dem verwendeten Lösungsmittel in der Art, dass z.B. die hydrophilen Reste der Substanz nach außen weisen und die hydrophoben Reste zueinander nach innen ausgerichtet sind oder umgekehrt.

Orientieren sich zwei Schichten der Substanz in dem oben beschriebenen Sinne, so spricht man von einer Einfachmembran, während bei einer Übereinanderordnung von zwei weiteren Schichten diese lamellare Struktur als Doppelmembran bezeichnet wird. Gemäß dem vorliegenden Prinzip können noch weitere Schichten an die bereits vorliegenden (Doppel)-Membran assoziiert werden, was zu einem Mehrfachmembranaufbau führt. Gemäß der vorliegenden Erfindung kann die Membran als Einfachmembran, als Doppelmembran oder auch als Mehrfachmembran vorliegen.

Unter dem "wash out" Effekt wird ein Absenken der Hautfeuchtigkeit nach Beenden der Applikation der Pflegezusammensetzung unterhalb des Ausgangswertes verstanden.

Gemäß der vorliegenden Erfindung sind körperidentische Fette, im Körper vorkommende Fette pflanzlichen Ursprungs.

### Ölphase:

Geeignete Komponenten, welche die Ölphase bilden können, können aus den polaren und unpolaren Ölen oder deren Mischungen gewählt werden.

Die Ölphase der erfindungsgemäßen Formulierungen wird vorteilhaft gewählt aus der Gruppe der Lecithine, (Mono-, Di-, Tri-)-Glyceride (wie z.B. der Fettsäuretriglyceride), Sphingolipide, Phospholipide, aus der Gruppe der Propylenglykole oder Butylenglykole, Fettsäureester, aus der Gruppe der natürlichen Wachse, tierischen und pflanzlichen Ursprungs, aus der Gruppe der Esteröle, aus der Gruppe der Dialkylether und Dialkylcarbonate, aus der Gruppe der verzweigten und unverzweigten Kohlenwasserstoffe und Wachse sowie aus der Gruppe der zyklischen und linearen Silikonöle.

Die Schaumformulierungen gemäß der vorliegenden Erfindung ermöglichen aufgrund des lamellaren Membranaufbaus und der daraus resultierenden strukturelle Ähnlichkeit zum Aufbau der interzellulären Schichtstruktur der Haut, insbesondere des *stratum corneums,* eine verbesserte Pflegewirkung der Formulierung. Durch den analogen Aufbau der lamellaren Struktur der Haut wird eine leichtere Integration der Membran in die Haut erzielt. Die Integration führt ebenfalls zu einer Verbesserung, insbesondere der Stabilisierung und der Wiederherstellung, der Hautbarriere. Eine intakte Hautbarriere schützt die Haut vor zu hohem Feuchtigkeitsverlust. Eine Verbesserung der Hautbarriere kann ebenfalls eine verbesserte Hautglättung bewirken und den "wash-out" Effekt verringern, wodurch vorteilhaft ein besserer Langzeiteffekt im Vergleich zu herkömmlichen Schaumformulierungen erzielt wird.

Bevorzugte Schaumformulierungen der vorliegenden Erfindung verwenden "hautähnliche" Bestandteile, um die Ähnlichkeit der lamellar angeordneten Membran, die in der Schaumformulierung vorliegt, mit der Haut zu erzeugen. Besonders bevorzugte Ausführungsformen ersetzen dabei z.B. die in der Hornschicht vorliegenden natürlichen Glyceride (die Haut enthält überwiegend ein Gemisch aus Di- und Triglyceriden) durch z.B. (pflanzliche) Triglyceride, das Squalen durch z.B. das weniger oxidationsempfindliche Squalan, Ceramide durch Ceramide 3 (aus Hefe), Cholesterol durch (pflanzliche) Phytosterole und die Phospholipide durch (pflanzliche) Phospholipide ersetzt.

Die membranbildende Substanz umfasst ein Lipid, welches hydriertes Lecithin umfasst.

In einer bevorzugten Schaumformulierung der Erfindung umfasst die membranbildende Substanz ferner ein Triglycerid. In einer besonders bevorzugten Schaumformulierung der Erfindung ist das Triglycerid Caprylsäure-/Caprinsäuretriglycerid.

Die bevorzugten erfindungsgemäßen Schaumformulierungen können ferner weitere Bestandteile wie z.B. Stabilisatoren wie z.B. Alkohole oder Glykole enthalten sein. Bevorzugt sind Glykole, insbesondere Propylenglykol, Caprylylglykol oder Mischungen davon.

In bevorzugten Schaumformulierungen der Erfindung können weitere Bestandteile wie z.B. Butyrospermum Parkii (Sheabutter), Squalan, Gylceride, Ceramide, bevorzugt Ceramid 3 oder Mischungen der vorgenannten umfassen.

Eine bevorzugte Schaumformulierung der Erfindung umfasst eine im Wesentlichen emulgatorfreie Emulsion. Eine besonders bevorzugte Schaumformulierung der Erfindung ist emulgatorfrei.

Basiscremes, die auf die oben beschriebenen "hautähnlichen" Bestandteile zurückgreifen, sind in der Technik auch als DMS® Basiscremes bekannt.

Die DMS® Basiszusammensetzungen können die folgenden Bestandteile Caprylsäure-/Caprinsäuretriglycerid, Sheabutter, Squalan, Ceramid 3, hydriertes Lecithin, Palm Glyceride, Persea Gratissima, Palmöl (Elaesis Guineensis) aufweisen.

Als Stabilisatoren können z.B. Alkohole oder Glykole wie z.B. Pentylenglykol, Caprylylglykol oder Mischungen davon in den DMS® Zusammensetzungen eingesetzt werden.

Eine kommerziell erhältliche DMS® Basis umfasst Caprylsäure-/Caprinsäuretriglycerid, Sheabutter, Squalan, Ceramid 3, hydriertes Lecithin sowie Pentylenglykol.

Eine weitere kommerziell erhältliche DMS® Basis umfasst
Caprylsäure-/Caprinsäuretriglycerid, Sheabutter, Squalan, Ceramid 3, hydriertes Lecithin sowie Alkohol.

Eine weitere kommerziell erhältliche DMS® Basis umfasst Caprylsäure-/Caprinsäuretriglycerid, Sheabutter, Squalan, Ceramid 3, hydriertes Lecithin, Persea Gratissima sowie Caprylylglykol.

Eine weitere kommerziell erhältliche DMS® Basis umfasst Caprylsäure-/Caprinsäuretriglycerid, Sheabutter, Squalan, Ceramid 3, hydriertes Lecithin, Palmglyceride, Elaesis Guineensis sowie Pentylenglykol.

Eine bevorzugte DMS® Basis umfasst Caprylsäure-/Caprinsäuretriglycerid, Butyrospermum Parkii, Squalan, Ceramid 3, hydriertes Lecithin sowie Pentylenglykol.

Ein besonders bevorzugtes Caprylsäure-/Caprinsäuretriglycerid ist erhältlich unter der Bezeichnung Miglyol 812 der Firma Sasol und dessen Abmischung mit weiteren Öl und Wachskomponenten.

Weiterhin sind besonders bevorzugt das Caprylsäure-/Caprinsäuretriglycerid, erhältlich unter der Bezeichnung Miglyol 812 der Firma Sasol /Myritol 312 der Fa. Cognis.

Die erfindungsgemäßen Emulsionen enthalten bevorzugt von 5 bis 50 Gew.-% Ölphase, besonders bevorzugt 10 bis 35 Gew.-% und mehr bevorzugt 15 bis 35 Gew.-% Ölphase. Die Angaben werden jeweils auf das Gesamtgewicht der Emulsion ohne Treibgas bezogen.

Diese Cremezusammensetzungen werden insbesondere bei gereizter, trockener bis sehr trockener, sensitiver bis sehr sensitiver, allergischer und ekzemischer Haut eingesetzt.

Außerdem kann die Ölphase bevorzugt weitere Bestandteile wie z.B. Fettsäuren, insbesondere Stearinsäure oder Öle wie z.B. Cetiol V enthalten.

Bevorzugt wird bei den DMS-Konzentraten und bei den erfindungsgemäßen Formulierungen auf weitere (nicht körperidentische) übliche Hilfsstoffe wie Duftstoffe, Farbstoffe, komedogene Lipide (z.B. Mineralöle) und physiologische Emulgatoren verzichtet, da diese potenziell sensibilisierend sind und zu Irritationen der Haut führen können.

### Wasserphase:

Die Wasserphase kann kosmetische Hilfsstoffe enthalten, z.B. niedere Alkohole (z.B. Ethanol, Isopropanol), niedere Diole oder Polyole sowie deren Ether (z.B. Propylenglykol, Glycerin, Butylenglykol, Hexylenglykol und Ethylenglykol), Schaumstabilisatoren und Verdickungsmittel.

Geeignete Verdickungsmittel sind polymere Verdickungsmittel, die teilweise wasserlöslich oder zumindest in Wasser dispergierbar sind und in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie die Beweglichkeit des Wassers einschränken. Geeignete Polymere sind z.B.:
■ Abgewandelte Naturstoffe, wie Celluloseether (z.B. Hydroxypropylcelluloseether, Hydroxyethlylcellulose und Hydroxypropylmethylcelluloseether);
■ Natürliche Verbindungen, wie z.B. Agar-Agar, Carrageen, Polyosen, Stärke, Dextrine, Gelatine, Casein;
■ Synthetische Verbindungen wie z.B. Vinylpolymere, Polyether, Polyimine, Polyamide und Derivate der Polyacrylsäure; und
■ Anorganische Verbindungen wie z.B. Polykieselsäure und Tonmineralien.

Bevorzugt ist ein Celluloseether als Verdickungsmittel in der Formulierung der Erfindung enthalten. Besonderes bevorzugt ist Hydroxypropylmethylcellulose. Eine erfindungsgemäße, bevorzugte Hydroxypropylmethylcellulose ist Metolose 90SH100.

Ein weiteres bevorzugtes Verdickungsmittel ist Xanthan Gummi, insbesondere Keltrol® CG Xanthan Gummi.

Die Hydroxypropylmethylcellulose und Xanthan Gummi können ebenfalls nebeneinander in der erfindungsgemäßen Formulierung eingesetzt werden.

Die erfindungsgemäßen Emulsionen enthalten bevorzugt von 0,2 bis 3,0 Gew.-% Verdickungsmittel (bezogen auf das Trockengewicht des Verdickungsmittels und das Gesamtgewicht der Emulsion ohne Treibgas). Besonders bevorzugt sind 0,5 bis 2,5 Gew.-% Verdickungsmittel.

### Wirkstoffe:

Der enthaltene Wirkstoff kann unter allen oberflächig auf der Haut applizierbaren Wirkstoffen und Mischungen dieser gewählt werden. Der Wirkstoff kann kosmetisch oder pharmazeutisch wirken. Entsprechend erhält man kosmetische oder dermatologische (als Medizinprodukt oder Arzneimittelprodukt einzusetzende) Schaumformulierungen. Ferner kann die Formulierung zum Schutz der Haut vor Umwelteinflüssen dienen. Der Wirkstoff kann rein pflanzlich oder synthetisch sein. Die Gruppe der Wirkstoffe kann sich auch mit den anderen Inhaltstoffgruppen, wie z.B. der Ölkomponente, den Verdickungsmitteln oder den Feststoffemulgatoren, überschneiden. Beispielsweise können manche Ölkomponenten auch als Wirkstoffe dienen, wie z.B. Öle mit mehrfach ungesättigten Fettsäuren, oder Feststoffemulgatoren, wie z.B. partikuläres Titandioxid als UV-Filter dienen kann. Je nach Eigenschaftsprofil sind die Substanzen mehreren Gruppen zuzuordnen.

Wirkstoffe der erfindungsgemäßen Formulierungen werden vorteilhaft gewählt aus der Gruppe der Substanzen mit feuchtigkeitspendenden und barrierestärkenden Eigenschaften, wie z. B. Hydroviton, eine Nachbildung des NMF, Pyrrolidoncarbonsäure und deren Salze, Milchsäure und deren Salze, Glycerol, Sorbitol, Propylenglykol und Harnstoff, Substanzen aus der Gruppe der Proteine und Proteinhydrolysate wie z. B. Collagen, Elastin sowie Seidenprotein, Substanzen aus der Gruppe der Glucosaminoglucane, wie z. B. Hyaluronsäure, aus der Gruppe der Kohlenhydrate, wie z. B. Pentavitin, das in seiner Zusammensetzung dem Kohlehydratgemisch der menschlichen Hornschicht entspricht, und der Gruppe der Lipide und Lipidvorstufen wie beispielsweise die Ceramide. Weitere vorteilhafte Wirkstoffe können im Sinne der vorliegenden Erfindung ferner gewählt werden aus der Gruppe der Vitamine, wie z. B. Panthenol, Niacin, α-Tocopherol und seine Ester, Vitamin A sowie Vitamin C. Außerdem können die Wirkstoffe, gewählt aus der Gruppe der Antioxidantien z. B. Galate und Polyphenole, verwendet werden. Harnstoff, Hyaluronsäure und Pentavitin sind bevorzugte Substanzen.

Es ist ferner bevorzugt, dass als Wirkstoffe Substanzen mit hautberuhigender und regenerierender Wirkung eingesetzt werden, wie z. B. Panthenol, Bisabolol und Phytosterole.

Vorteilhafte Wirkstoffe im Sinne der vorliegenden Erfindung sind auch Pflanzen und Pflanzenextrakte. Hierzu gehören z.B. Algen, Aloe, Arnika, Bartflechten, Beinwell, Birke, Brennessel, Calendula, Eiche, Efeu, Hamamelis, Henna, Hopfen, Kamille, Mäusedorn, Pfefferminze, Ringelblume, Rosmarin, Salbei, grüner Tee, Teebaum, Schachtelhalm, Thymian und Walnuss sowie deren Extrakte.

Die erfindungsgemäßen Zubereitungen können ferner als Wirkstoffe Antimykotika und Antiseptika/Desinfizientia synthetischen oder natürlichen Ursprungs enthalten.

Weitere Wirkstoffe sind Glucocortikoide, Antibiotika, Analgetika, Antiphlogistika, Antirheumatika, Antiallergika, Antiparasitika, Antipruriginosa, Antipsoriatika, Retinoide, Lokalanästhetika, Venentherapeutika, Keratolytika, Hyperämisierende Substanzen, Koronartherapeutika (Nitrate/Nitro-Verbindungen), Virusstatika, Zytostatika, Hormone, Wundheilungsfördernde Wirkstoffe, z.B. Wachstumsfaktoren, Enzympräparate und Insektizide.

### Weitere Bestandteile der Emulsion:

Die Formulierungen können außerdem optional Farbstoffe, Perlglanzpigmente, Duftstoffe/Parfum, Lichtschutzfiltersubstanzen, Konservierungsmittel, Komplexbildner, Antioxidantien und Repellentien sowie pH-Wert Regulatoren enthalten.

Die erfindungsgemäßen Schaumformulierungen können neben den bereits oben genannten Bestandteilen weitere natürliche Fette, wie z. B. Sheabutter, Neutralöle, Olivenöl, Squalan, Ceramide und Feuchthaltesubstanzen enthalten, wie sie in der Technik üblich sind.

Die obige Aufzählung der einzelnen Bestandteile der Emulsion soll so verstanden werden, dass einzelne Beispielkomponenten aufgrund ihrer verschiedenen Eigenschaften auch mehreren Gruppen zugeordnet werden können.

### Treibgase:

Geeignete Treibgase sind z.B. N₂O, Propan, Butan und i-Butan, Die fertige Schaumformulierung enthält von etwa 5 bis etwa 15 Gew.-%, bevorzug etwa 10 Gew.-% Treibgas.

### Herstellungsverfahren:

Die erfindungsgemäßen Schaumformulierungen werden durch Bereitstellen einer Emulsion, bevorzugt vom Typ Öl-in-Wasser und Abfüllen der Emulsion und gegebenenfalls Beaufschlagen mit Treibgas in einen geeigneten Behälter wie z.B. einen Druckbehälter bereitgestellt. Alternativ zum Treibgas und Druckbehälter kann die Emulsion auch in einen anderen Behälter abgefüllt werden, der auch ohne Treibgas dafür geeignet ist, die Emulsion als Schaum abzugeben. Solche Systeme sind dem Fachmann bekannt.

Insbesondere werden die erfindungsgemäßen Emulsionen durch ein Verfahren hergestellt, das die folgenden Schritte umfasst:
(1) Bereitstellen einer Ölphase gegebenenfalls umfassend mindestens eine membranbildende Substanz, die in der Formulierung eine lamellare Membran ausbildet,
(2) Bereitstellen einer Wasserphase,
(3) Zusammengeben und Homogenisieren der beiden Phasen,
(4) Optional Hinzufügen mindestens einer oder mindestens einer weiteren membranbildenden Substanz,
(5) Optional Homogenisieren, um eine Emulsion zu erhalten,
   wobei in mindestens einem der Schritte (1) oder (4) mindestens eine membranbildende Substanz enthalten ist, die in der Formulierung eine lamellare Membran ausbildet.

Bevorzugt werden die Ölphase und die Wasserphase jeweils bei einer Temperatur im Bereich von etwa 40 bis etwa 90 °C vermischt und homogenisiert, besonders bevorzugt ist ein Temperaturbereich von etwa 60 bis etwa 80 °C, mehr bevorzugt eine Temperatur von etwa 70 °C.

Für das Homogenisieren kann jedes Mittel oder Verfahren, welches in der Technik bekannt ist, verwendet werden. Bevorzugt werden die Phasen mit einem hochtourigen Rührwerk homogenisiert.

In einem bevorzugten Herstellungsverfahren wird die Ölphase in die Wasserphase eingerührt und homogenisiert. Falls erforderlich wird die Emulsion unter Rühren auf Raumtemperatur abgekühlt. In einem besonders bevorzugten Verfahren wird zu dieser Mischung eine geeignete Menge eines DMS® Konzentrats hinzugegeben und das Konzentrat in die vorliegende Emulsion eingearbeitet.

Das DMS® Konzentrat kann bereits vor dem Homogenisieren mit der Wasserphase in die Ölphase gegeben werden oder nach dem Homogenisieren der Öl- und Wasserphase zu der Mischung hinzugegeben werden. Es ist bevorzugt, dass das DMS® Konzentrat nach dem ersten Homogenisierungsschritt zu der Mischung hinzugegeben und die Mischung homogenisiert wird.

Wenn die Emulsion ein Verdickungsmittel umfasst, umfasst das Verfahren vorteilhaft die folgenden weiteren Schritte:
(6) Bereitstellen einer wässrigen Verdickungsmittellösung
(7) Vermischen der Verdickungsmittellösung mit der Emulsion

Bevorzugt wird die erfindungsgemäße Emulsion mit etwa 10 Gew.-% Treibgas beaufschlagt.

### Verwendungen:

Die Schaumformulierungen der vorliegenden Erfindung können für alle kosmetischen und dermatologischen (als Medizinprodukt oder Arzneimittel) Zwecke eingesetzt werden. Zum Beispiel könne die Formulierungen als Hautpflegemittel oder Hautreinigungsmittel eingesetzt werden. Sie können ferner als Träger für Wirkstoffe dienen und im medizinisch dermatologischen Bereich eingesetzt werden. Insbesondere können die Formulierungen als Sonnenschutzmittel eingesetzt werden.

### Beispiele:

### Zusammensetzung der Schaumformulierung:

a) Wasserphase
Die Wasserphase wird durch Mischen der Bestandteile hergestellt.

| Bestandteil | Menge |
|---|---|
| HPMC (Metolose 90SH100) | 1,5 g |
| Xanthan Gummi (Keltrol® CG) | 0,5 g |
| Wasser | 78 g |

b) Ölphase

### Beispiel 1:

| Bestandteil | Beispiel 1 |
|---|---|
| DMS Konzentrat | 5 g |
| Miglyol 812 | 14 g |
| Stearinsäure | 1 g |
| Wasserphase | ad 100 g |

### Beispiel 2:

| Bestandteil | Beispiel 2 |
|---|---|
| DMS Konzentrat | 5 g |
| Miglyol 812 | 14 g |
| Stearinsäure | 1 g |
| Cetiol V | 5 g |
| Wasserphase | ad 100 g |

Die Stearinsäure wird unter Erwärmen auf ca. 70 °C in Miglyol 812 (Beispiel 1) bzw. in der Mischung aus Miglyol 812 und Cetiol V (Beispiel 2) gelöst.

Diese Ölphase wird in die Wasserphase eingerührt und mit einem hochtourigen Rührwerk homogenisiert. Die resultierende Emulsion wird unter Rühren auf Raumtemperatur abgekühlt und das DMS®-Konzentrat mit einem hochtourigen Rührwerk eingearbeitet.

Das verwendete DMS® Konzentrat weist die folgende INCI-Bestandteile auf:
Aqua (and) Hydrogenated Lecithin (and) Caprylic/Capric Triglyceride(and) Pentylene Glycol (and) Butyrospermum Parkii (and) Glycerin (and) Squalane (and) Ceramide 3

### Herstellung der Schaumformulierung:

90 g der so hergestellte Emulsion werden in Aerosolbehälter abgefüllt und nach deren Verschließen mit einem Ventildeckel mit 10 g Treibmittel beaufschlagt.

## Patentansprüche

1. Schaumformulierung umfassend eine Emulsion, welche eine Ölphase und eine Wasserphase umfasst, wobei die Ölphase mindestens eine membranbildende Substanz umfasst, die in der Schaumformulierung eine lamellar angeordnete Membran ausbildet, die als mindestens eine membranbildende Substanz ein Lipid umfasst, wobei das Lipid hydriertes Lecithin umfasst.

2. Schaumformulierung gemäß Anspruch 1, wobei die Emulsion eine Öl-in-Wasser Emulsion ist.

3. Schaumformulierung gemäß einem der Ansprüche 1-2, wobei das Lipid ferner ein Triglycerid umfasst.

4. Schaumformulierung gemäß Anspruch 3, wobei das Triglycerid Caprytsäure/Caprinsäuretriglycerid umfasst.

5. Schaumformulierung gemäß einem der Ansprüche 1-4, wobei die Emulsion ferner mindestens ein Verdickungsmittel umfasst, welches bevorzugt ausgewählt wird aus der Gruppe bestehend aus Hydroxypropylmethylcellulose, Xanthan Gummi und Mischungen der vorgenannten.

6. Schaumformulierung gemäß einem der Ansprüche 1-5, wobei die Emulsion ferner einen Stabilisator umfasst, wobei der Stabilisator Pentylenglykol ist.

7. Schaumformulierung gemäß einem der Ansprüche 1-6, wobei die Emulsion ferner weitere übliche Bestandteile wie Sheabutter, Glycerin, Squalan, Ceramid, bevorzugt Ceramid 3 oder Mischungen der vorgenannten umfasst.

8. Schaumformulierung gemäß einem der Ansprüche 1-7, wobei die Emulsion ferner weitere übliche Bestandteile wie Öle und fettende Substanzen umfasst.

9. Schaumformulierung gemäß einem der Ansprüche 1-8, wobei die Emulsion ferner mindestens einen Wirkstoff umfasst.

10. Schaumformulierung gemäß Anspruch 9, wobei der Wirkstoff ausgewählt wird aus der Gruppe bestehend aus Hydroviton, Pyrrolidoncarbonsäure und deren Salze, Milchsäure und deren Salze, Glycerol, Sorbitol, Propylenglykol, Harnstoff, Collagen, Elastin, Seidenprotein, Hyaluronsäure, Pentavitin, Ceramide, Panthenol, Niacin, α-Tocopherol und seine Ester, Vitamin A, Vitamin C, Galate, Polyphenole, Panthenol, Bisabolol, Phytosterole, Glucocortikoide, Antibiotika, Analgetika, Antiphlogistika, Antirheumatika, Antiallergika, Antiparasitika, Antipruriginosa, Antipsoriatika, Retinoide, Lokalanästhetika, Venentherapeutika, Keratolytika, Hyperämisierende Substanzen, Koronartherapeutika (Nitrate/Nitro-Verbindungen), Virusstatika, Zytostatika, Hormone, Wundheilungsfördernde Wirkstoffe, Wachstumsfaktoren, Enzympräparate, Insektizide und Pflanzenmaterial wie, bzw. Pflanzenextrakte von, Algen, Aloe, Arnika, Bartflechten, Beinwell, Birke, Brennnessel, Calendula, Eiche, Efeu, Hamamelis, Henna, Hopfen, Kamille, Mäusedorn, Pfefferminze, Ringelblume, Rosmarin, Salbei, grüner Tee, Teebaum, Schachtelhalm, Thymian und Walnuss oder Mischungen davon, ausgewählt ist.

11. Schaumformulierung gemäß Anspruch 1-10, wobei das Lipid körperidentische Fette umfasst.

12. Schaumformulierung gemäß einem der Ansprüche 1-10, wobei die Formulierung eine Schaumcreme ist.

13. Verwendung einer Schaumformulierung gemäß einem der Ansprüche 1-12 als Hautpflegemittel.

14. Verwendung einer Schaumformulierung gemäß einem der Ansprüche 1-12 als Hautreinigungsmittel.

15. Verwendung einer Schaumformulierung gemäß einem der Ansprüche 1-12 zur Herstellung eines Kosmetikums, Medizinproduktes oder Arzneimittels.

16. Verfahren zur Herstellung einer Schaumformulierung gemäß einem der vorstehenden Ansprüche, umfassend die folgenden Schritte:
a) Herstellen einer Emulsion bevorzugt vom Typ Öl in Wasser
b) Abfüllen der Emulsion und Treibgas in einen Druckbehälter, oder
c) Abfüllen der Emulsion in einen anderen als einen Druckbehälter, der bei Abgabe der Emulsion einen Schaum erzeugt.

17. Verfahren gemäß Anspruch 16, wobei das Herstellen der Emulsion die Schritte umfasst:
(1) Bereitstellen einer Ölphase gegebenenfalls umfassend mindestens eine membranbildende Substanz, die in der Formulierung eine lamellare Membranen ausbildet,
(2) Bereitstellen einer Wasserphase,
(3) Zusammengeben und Homogenisieren der beiden Phasen,
(4) Optional Hinzufügen mindestens einer oder mindestens einer weiteren membranbildenden Substanz,
(5) Optional Homogenisieren, um eine Emulsion zu erhalten,
wobei in mindestens einem der Schritte (1) oder (4) mindestens eine membranbildende Substanz umfasst ist, die in der Formulierung eine lamellare Membran ausbildet.

18. Verfahren gemäß Anspruch 17, wobei die Ölphase und die Wasserphase bei einer Temperatur zwischen etwa 40 bis etwa 90 °C, bevorzugt zwischen etwa 60 bis etwa 80 °C, mehr bevorzugt etwa 70 °C homogenisiert werden.

19. Verfahren gemäß Anspruch 17 oder 18, wobei die Emulsion ein Verdickungsmittel umfasst, ferner umfassend die Schritte:
(6) Bereitstellen einer wässrigen Verdickungsmittellösung
(7) Mischen der Verdickungsmittellösung mit der Emulsion.

20. Verfahren gemäß einem der Ansprüche 18 bis 19, wobei die Schaumformulierung 10 Gew-% Treibgas enthält.

## Claims

1. Foam formulation comprising an emulsion which comprises an oil phase and a water phase, wherein the oil phase comprises at least one membrane-forming substance forming a lamellar membrane in the foam formulation, the at least one membrane-forming substance comprises a lipid, wherein the lipid comprises hydrogenated lecithin.

2. Foam formulation according to claim 1, wherein the emulsion is an oil-in-water emulsion.

3. Foam formulation according to anyone of claims 1-2, wherein the lipid comprises a triglyceride.

4. Foam formulation according to claim 3, wherein the triglyceride comprises caprylic acid/caprinic acid triglyceride.

5. Foam formulation according to any one of claims 1-4, wherein the emulsion further comprises at least one thickening agent preferably selected from the group consisting of hydroxypropylmethyl cellulose, xanthan, and mixtures thereof.

6. Foam formulation according to any one of claims 1-5, wherein the emulsion further comprises a stabilizer, wherein the stabilizer is pentylene glycol.

7. Foam formulation according to any one of claims 1-6, wherein the emulsion further comprises conventional components such as shea butter, glycerol, squalan, ceramide, preferably ceramide 3 or mixtures of the aforementioned.

8. Foam formulation according to any one of claims 1-7, wherein the emulsion further comprises conventional components such as oils and lubricating substances.

9. Foam formulation according to anyone of claims 1-8, wherein the emulsion further comprises at least one active agent.

10. Foam formulation according to claim 9, wherein the active agent is selected from the group consisting of hydroviton, pyrrolidone carbonic acid and salts thereof, lactic acid and salts thereof, glycerol, sorbitol, propylene glycol, urea, collagen, elastin, silk protein, hyaluronic acid, pentavitin, ceramides, panthenol, niacin, α-tocopherol and esters thereof, vitamin A, vitamin C, galates, polyphenols, panthenol, bisabolol, phytosteroles, glycocorticoides, antibiotics, analgetics, antiphlogistics, antirheumatics, antiallergics, antiparasitatics, antipruriginosics, antipsoriatics, retinoids, local anaesthetics, therapeutic agents for the veins, ceratolytics, hyperemisic compounds, coronary therapeutics (nitrates/nitro-compounds), virus statics, cytostatics, hormones, agents promoting wound healing, growth factors, enzyme preparations, insecticides and plant material such as e.g. plant extracts of algae, aloe, arnica, barber's rash, comfrey, birch, stinging nettle, calendula, oak, ivy, witch hazel, henna, hops, camomile, ruscus, peppermint, marigold, rosemary, sage, green tea, tea tree, horsetail, thyme and walnut or mixtures thereof.

11. Foam formulation according to anyone of claims 1-10, wherein the lipid comprises bioidentical fats.

12. Foam formulation according to any one of claims 1-11, wherein the formulation is a foam cream.

13. Use of a foam formulation according to any one of claims 1-12, as a skin care agent.

14. Use of a foam formulation according to any one of claims 1-12, as a skin cleaning agent.

15. Use of foam formulation according to any one of claims 1-12 for the manufacture of a cosmetic agent, medical agent of pharmaceutical composition.

16. Method for producing a foam formulation according to any one of the preceding claims, comprising the following steps:
a) Producing an emulsion, preferably of the oil-in-water type,
b) Filling the emulsion and propellant into a pressurized container, or
c) Filling the emulsion into a container other than a pressurized container that upon dispensing of the emulsion generates a foam.

17. Method according to claim 16, wherein producing of the emulsion comprises the steps:
1) Providing an oil phase optionally comprising at least one membrane-forming substance that forms a lamellar membrane in the formulation,
2) Providing a water phase,
3) Adding and homogenizing of both phases,
4) Optionally adding at least one or at least one further membrane-forming substance,
5) Optionally homogenizing, in order to obtain an emulsion,
wherein in at least one of the steps (1) or (4) at least one membrane-forming substance is comprised that forms a lamellar membrane in the formulation.

18. Method according to claim 17, wherein the oil phase and the water phase are homogenized at a temperature between about 40 up to about 90°C, preferably between about 60 up to about 80°C, more preferably at a temperature of about 70°C.

19. Method according to any one of claims 17 or 18, wherein the emulsion comprises a thickening agent, further comprising the steps:
6) Providing an aqueous solution of thickening agent,
7) Mixing the solution of thickening agent with the emulsion.

20. Method according to any one of claims 18-19, wherein the foam formulation contains 10 weight percent propellant.

## Revendications

1. Formulation de mousse comprenant une émulsion, qui renferme une phase huileuse et une phase aqueuse, la phase huileuse comprenant au moins une substance formant membrane, qui forme dans la formulation de mousse une membrane lamellaire, laquelle renferme un lipide en tant qu'au moins une substance formant membrane, le lipide comprenant de la lécithine hydratée.

2. Formulation de mousse selon la revendication 1, dans laquelle l'émulsion est une émulsion huile dans l'eau.

3. Formulation de mousse selon l'une des revendications 1 - 2, dans laquelle le lipide renferme en outre un triglycéride.

4. Formulation de mousse selon la revendication 3, dans laquelle le triglycéride renferme du triglycéride d' acide caprique/d'acide caprylique.

5. Formulation de mousse selon l'une des revendications 1 - 4, dans laquelle l'émulsion renferme en outre au moins un épaississant, sélectionné de préférence parmi le groupe constitué d'hydroxypropylméthylcellulose, de gomme xanthane et de mélanges des substances précitées.

6. Formulation de mousse selon l'une des revendications 1 - 5, dans laquelle l'émulsion renferme en outre un stabilisant, le stabilisant étant du pentylène glycol.

7. Formulation de mousse selon l'une des revendications 1 - 6, dans laquelle l'émulsion renferme en outre d'autres constituants courants, tels que beurre de Karité, glycérine, squalane, céramide, de préférence céramide 3, ou des mélanges des substances précitées.

8. Formulation de mousse selon l'une des revendications 1 - 7, dans laquelle l'émulsion renferme en outre d'autres constituants courants, tels que des huiles et substances lubrifiantes.

9. Formulation de mousse selon l'une des revendications 1 - 8, dans laquelle l'émulsion renferme en outre au moins une substance active.

10. Formulation de mousse selon la revendication 9, dans laquelle la substance active est sélectionnée parmi le groupe constitué d'Hydroviton, d'acide pyrrolidone carboxylique et de ses sels, d'acide lactique et de ses sels, de glycérol, de sorbitol, de propylène glycol, d'urée, de collagène, d'élastine, de protéine de soie, d'acide hyaluronique, de Pentavitine, de céramide, de panthénol, de Niacine, d' α-tocophérol et de ses esters, de vitamine A, de vitamine C, de galate, de polyphénols, panthénol, de bisabolol, de phytostérols, de glucocorticoïdes, d'antibiotiques, d'analgésiques, d'antiphlogistiques, d'antirhumatismaux, d'antialler-giques, d'antiparasitaires, d'antiprurigineux, d'anti-psoriatiques, de rétinoïdes, d'anesthésiants locaux, de thérapeutiques veineux, de kératolytiques, de substances antiémétiques, de thérapeutiques coronariens (nitrates/dérivés nitrés), de virostatiques, de cytostatiques, d'hormones, de substances actives cicatrisantes, de facteurs de croissance, de préparations enzymatiques, d'insecticides et matières végétales, telles que, par exemple, extraits végétaux d'algues, aloès, arnica, lichen, consoude, bouleau, ortie, calendula, chêne, lierre, hamamélis, henné, houblon, camomille, fragon, menthe, souci, romarin, sauge, thé vert, théier, prêle, thym et noix ou de mélanges de ces derniers.

11. Formulation de mousse selon l'une des revendications 1 - 10, dans laquelle le lipide renferme de la graisse bio-identique.

12. Formulation de selon l'une des revendications 1 - 10, dans laquelle la formulation est une crème moussante.

13. Utilisation d'une formulation de mousse selon l'une des revendications 1 - 12 en tant que produit principal de soins.

14. Utilisation d'une formulation de mousse selon l'une des revendications 1 - 12 en tant que nettoyant pour la peau.

15. Utilisation d'une formulation de mousse selon l'une des revendications 1 - 12 pour la préparation d'un cosmétique, d'un produit pharmaceutique ou d'un médicament.

16. Procédé de préparation d'une formulation de mousse selon l'une des revendications précédentes, comprenant les étapes suivantes :
a) préparation d'une émulsion, de préférence du type huile dans l'eau
b) remplissage de l'émulsion et du gaz propulseur dans un récipient sous pression, ou
c) remplissage de l'émulsion dans un récipient autre qu'un récipient sous pression, qui génère une mousse lors de la sortie de l'émulsion.

17. Procédé selon la revendication 16, dans lequel la préparation de l'émulsion comprend les étapes suivantes :
(1) préparation d'une phase huileuse comprenant éventuellement au moins une substance formant membrane, qui forme dans la formulation une membrane lamellaire,
(2) préparation d'une phase aqueuse,
(3) addition et homogénéisation des deux phases,
(4) ajout optionnel d'au moins une ou d'au moins une autre substance membranogène,
(5) homogénéisation optionnelle, pour obtenir une émul-sion,
au moins une substance formant membrane étant comprise dans au moins l'une des étapes (1) ou (4), laquelle substance forme dans la formulation une membrane lamellaire.

18. Procédé selon la revendication 17, dans lequel la phase huileuse et la phase aqueuse sont homogénéisées sous une température d'environ 40 à 90°C, de préférence d'environ 60 à 80°C, plus préférentiellement d'environ 70°C.

19. Procédé selon l'une des revendications 17 et 18, l'émulsion renfermant un épaississant, comprenant en outre les étapes :
(6) préparation d'une solution aqueuse d'épaississant
(7) mélange de la solution d'épaississant avec l'émulsion.

20. Procédé selon l'une des revendications 18 à 19, dans lequel la formulation de mousse renferme 10% en poids de gaz propulseur.
